# EUROPEAN PATENT SPECIFICATION

(11) **EP 0 451 626 B1**
(45) Date of publication and mention of the grant of the patent: **10.09.1997**
(21) Application number: 91104888.2
(22) Date of filing: 27.03.1991
(51) Int. Cl.: A61K 31/55

(54) **The use of benzodiazepine compounds for preparing a pharmaceutical composition against CNS disorders related to the excitatory aminoacids**
Verwendung von Benzodiazepinverbindungen zur Herstellung eines Arzneimittels gegen mit excitatorischen Aminosäuren verbundene Zentralnervensystem-Störungen
Utilisation de benzodiazepines dans la préparation d'une composition pharmaceutique contre les troubles du SNC en relation avec les acides aminées excitateurs

(30) Priority: 13.04.1990 US 509284
(43) Date of publication of application: 16.10.1991
(73) Proprietor: NEUROSEARCH A/S, DK-2600 Glostrup (DK)
(72) Inventor: Jensen, Leif Helth, DK-1573 Copenhagen V (DK); Drejer, Jorgen, DK-3500 Vaerlose (DK); Wätjen, Frank, DK-2730 Herlev (DK)
(74) Representative: Grünecker, Kinkeldey, Stockmair & Schwanhäusser Anwaltssozietät

(56) References cited:
- EP-A- 0 100 906
- EP-A- 0 331 871
- BR. J. PHARMACOL., vol. 98, no. 3, 1989, pages 1050-1054, The Macmillan Press Ltd.; J.-L. MOREAU et al.: "Convulsions induced by centrally administered NMDA in mice: effects of NMDA antagonists, benzodiazepines, minor tranquilizers and anticonvulsants"
- EUROPEAN JOURNAL OF PHARMACOLOGY, vol. 156, no. 1, 1988, pages 169-172, Elsevier Science Publishers B.V. (Biomedical Division); M.-C. POTIER et al: "Demonstration of the partial agonist profiles of Ro 16-6028 and Ro 17-1812 in mice in vivo"
- SOC. NEUROSCI. ABSTRACTS, vol. 10, no. 2, 1984, pages 1005, abstrqct no. 293. 19; G. DELBARRE et al.: "Reversal of induced ischemic neurologic deficit in gerbils by L-cycloserin, agonist and antagonist of benzodiazepines (BZD) receptors"
- ANESTHESIOLOGY, vol. 71, no. 3A, September 1989, abstract no. A126; A. FORSTER et al.: "Survival during severe hypoxia after flumazenil and an association of flumazenil and midazolam in mice"
- STN INTERNATIONAL, Karlsruhe, DE, File Registry. Registry numbers 90450-01-4, 84379-13-5, 78771-13-8, 78755-81-4, American Chemical Society

## Description

The present invention relates to a novel use of benzodiazepine compounds for preparing pharmaceutical compositions.

It is an object of the present invention to provide a novel use of benzodiazepine compounds.

The benzodiazepine compounds useful for preparing the pharmaceutical compositions have the formula I 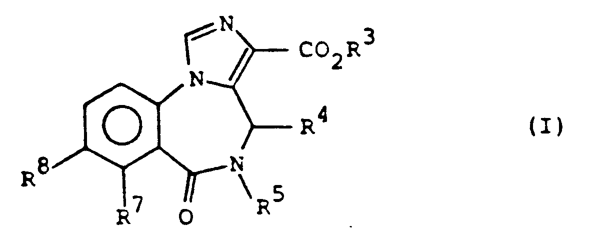 wherein
R³ is hydrogen;
R⁷ and R⁸ are independently hydrogen, halogen, CF₃, CN, NO₂, NH₂, C₁₋₄-alkyl or C₁₋₄-alkoxy; and
R⁴ is hydrogen and R⁵ is hydrogen or C₁₋₇-alkyl; or R⁴ and R⁵ together signify (CH₂)ₙ wherein n is an integer of 2-3.

The pharmaceutical compositions prepared according to the present invention are extremely useful in the treatment of central nervous system disorders related to the excitatory amino acids.

Examples of compounds having the formula above are for example:
7-Fluor-5,6-dihydro-5-methyl-6-oxo-4H-imidazo[1,5-a][1,4]benzodiazepine-3-carboxylate,
7-Chlor-5,6-dihydro-5-methyl-6-oxo-4H-imidazo[1,5-a][1,4]benzodiazepine-3-carboxylate,
(S)-8-Chlor-11,12,13,13a-tetrahydro-9-oxo-9H-imidazo[1,5-a]pyrrolo[2,1-c][1,4]benzodiazepine-1-carboxylate,
(R,S)-11,13a-Dihydro-9-oxo-9H-imidazo[1,5-a]-pyrrolo[2,1-c][1,4]benzodiazepine-1-carboxylate;
(R,S)-8-Fluor-11,13a-dihydro-9-oxo-9H-imidazo[1,5a]pyrrolo[2,1-c][1,4]benzodiazepine-1-carboxylate,
5,6-Dihydro-5-methyl-6-oxo-4H-imidazo[1,5-a][1,4]benzodiazepine-3-carboxylate,
5,6-dihydro-5-methyl-6-oxo-4H-imidazo[1,5-a][1,4]benzodiazepine-3-carboxylate,
5,6-dihydro-5-methyl-6-oxo-4H-imidazo[1,5-a][1,4]benzodiazepine-3-carboxylate,
8-fluor-5,6-dihydro-5-methyl-6-oxo-4H-imidazo[1,5a][1,4]benzodiazepine-3-carboxylate,
8-fluor-5,6-dihydro-5-methyl-6-oxo-4H-imidazo[1,5-a][1,4]benzodiazepine-3-carboxylate,
5,6-Dihydro-5,7-dimethyl-6-oxo-4H-imidazo[1,5-a][1,4]benzodiazepine-3-carboxylate,
(S)-11,12,13,13a-Tetrahydro-9-oxo-9H-imidazo[1,5-a]pyrrolo[2,1-c[[1,4]benzodiazepine-1-carboxylate,
(S)-11,12,13,13a-tetrahydro-9-oxo-9H-imidazo[1,5-a]pyrrolo[2,1-c][1,4]benzodiazepine-1-carboxylate,
(S)-11,12,13,13a-tetrahydro-9-oxo-9H-imidazo[1,5-a]pyrrolo[2,1-c][1,4]benzodiazepine-1-carboxylate,
(S)-7-Fluor-11,12,13,13a-tetrahydro-9-oxo-9H-imidazo[1,5a]pyrrolo[2,1-a][1,4]benzodiazepine-1-carboxylate,
(S)-11,12,13,13a-Tetrahydro-8-methyl-9-oxo-9H-imidazo[1,5a]pyrrolo[2,1-a][1,4]benzodiazepine-1-carboxylate.

The compounds can be prepared as described in
USP 4,316,839; USP 4,352,817; USP 4,386,028; USP 4,352,816; USP 4,352,818; USP 4,353,827; and USP 4,489,003.

It has been found that the compounds of formula I wherein R³ is an alkyl group are metabolized to the corresponding compounds wherein R³ is H, and it has been found that the compounds wherein R³ is H are more potent than their corresponding ester analogues. Thus it seems highly likely the biological active chemical entities of formula I all are compounds wherein R³ is H.

### Biological Activity

The compounds of formula I exhibit Valuable biological properties.

For example the compounds exhibit strong pharmacological in vivo ATPA and quisqualate antagonizing effects demonstrating the utility as novel orally bioavailable quisqualate or kainate antagonists, which makes them useful in the treatment of for example psychosis, anoxia, ischemia and migraine.

### Biological testing

The above mentioned tests are performed as described in more detail below and as based upon the principles also described hereinafter.

### ATPA-induced rigidity

The selective quisqualate receptor agonist ATPA induce rigidity in female NMRI mice at doses between 3 and 15 mg/kg and clonic-tonic seizures and death, probably due to respiratory arrest, at doses between 15 and 40 mg/kg after intravenous (i.v.) administration.

### Method

ATPA ((RS)-α-amino-3-hydroxy-5-tert-butyl-4-isoxazolepropionic acid) was dissolved in distilled water and test compound was dissolved in a polyoxyl 40 hydrogenated castor oil (5% Cremophor RH™ 40 (BASF)).

Test compound was administered either i.v. 5, 30 or 120 min before or p.o. 30 min before an i.v. administration of 15 mg/kg of ATPA to 8 female NMRI mice per dose and the number of mice experiencing rigidity 5 min later was noted. An ED₅₀ value was calculated from at least three doses of test compound as the dose inhibiting 50% of the mice from having rigidity.

### Quisqualate-induced clonic seizures

Quisqualate given icv to DBA/2 mice induce clonic seizures which can be inhibited by both NMDA and non-NMDA receptor antagonists after icv administration.

### Method

Test compound was given i.v. 5 min before a 20 µg icv administration of quisqualate to 10 male DBA/2 mice (weighing 10-12 g) per dose. The number of mice experiencing clonic seizures within the next 2 min was noted. An ED₅₀ value was calculated as the dose inhibiting 50% of the mice from having clonic seizures.

The table below presents some data obtained by testing selected compounds.

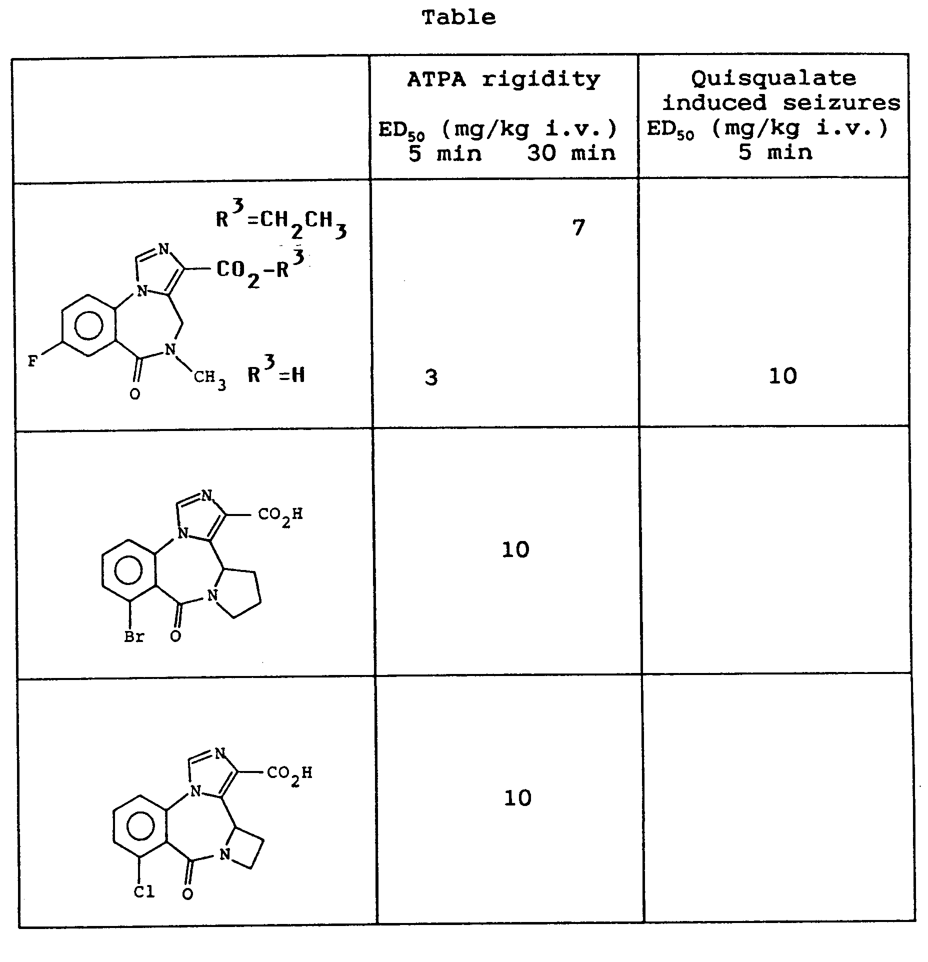

### Pharmaceutical Compositions

The compounds of formula I, together with a conventional adjuvant, carrier, or diluent, may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed. Tablets containing ten (10) milligrams of active ingredients or, sore broadly, one (1) to one hundred (100) milligrams, per tablet, are accordingly suitable representative unit dosage forms.

### Method of Treating

The pharmaceutical compositions containing compounds of formula I are extremely useful in the treatment of central nervous system disorders related to the excitatory amino acids. The pharmaceutical compositions may accordingly be administered to a subject, including a human, in need of treatment, alleviation, or elimination of an indication associated with dysfunctions related to the excitatory amino acids. This includes especially psychosis, anoxia, ischemia and migraine.

## Claims (Claims for the following Contracting State(s): AT, BE, CH, DE, FR, GB, GR, IT, LI, LU, NL, SE)

1. The use of a benzodiazepine compound having the formula 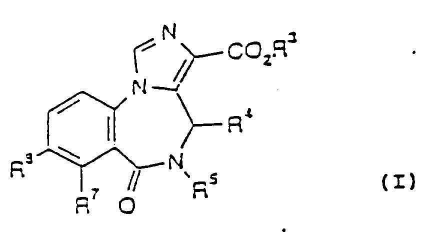 wherein
R³ is hydrogen
R⁷ and R⁸ are independently hydrogen, halogen, CF3, CN, NO₂, NH₂, C₁₋₄-alkyl or C₁₋₄-alkoxy; and
R⁴ is hydrogen and R⁵ is hydrogen or C₁₋₇-alkyl; or R⁴ and R⁵ together signify (CH₂)ₙ wherein n is an integer of 2-3, as the active ingredient for preparing a pharmaceutical composition for treating a central nervous system disorder responsive to antagonization of excitatory amino acids acting via the quisqualate or kainate receptors.

2. The use according to claim 1 wherein the active ingredient is 8-fluor-5,6-dihydro-5-methyl-6-oxo-4H-imidazo[1,5-a] [1,4]benzodiazepine-3-carboxylate.

3. The use according to claim 1 wherein the active ingredient is 8-brom-11,12,13,13a-tetrahydro-9-oxo-9H-imidazo[1,5-a] pyrrolo[2,1-c][1,4]benzodiazepine-1-carboxylate.

4. The use according to claim 1 wherein the active ingredient is 12,12a-dihydro-8-chloro-9-oxo-9H,11H-azeto[2,1-c] imidazo[1,5-a][1,4]benzodiazepine-1-carboxylate.

## Claims (Claims for the following Contracting State(s): ES)

1. A process for preparing a pharmaceutical composition for treating a central nervous system disorder responsive to antagonization of excitatory amino acids acting via the quisqualate or kainate receptors, comprising the step of placing the compound having the formula: 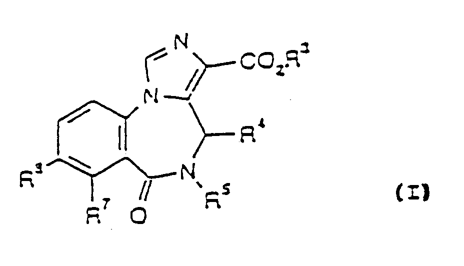 wherein
R³ is hydrogen
R⁷ and R⁸ are independently hydrogen, halogen, CF₃, CN, NO₂, NH₂, C₁₋₄-alkyl or C₁₋₄-alkoxy; and
R⁴ is hydrogen and R⁵ is hydrogen or C₁₋₇-alkyl; or R⁴ and R⁵ together signify (CH₂)ₙ wherein n is an integer of 2-3, together with a conventional adjuvant, carrier or diluent into the form of a pharmaceutical composition or unit dosage thereof.

2. The process according to claim 1 wherein the active ingredient is 8-fluor-5,6-dihydro-5-methyl-6-oxo-4H-imidazo[1,5-a][1,4]benzodiazepine-3-carboxylate.

3. The process according to claim 1 wherein the active ingredient is 8-brom-11,12,13,13a-tetrahydro-9-oxo-9H-imidazo[1,5-a]pyrrolo[2,1-c][1,4]benzodiazepine-1- carboxylate.

4. The process according to claim 1 wherein the active ingredient is 12,12a-dihydro-8-chloro-9-oxo-9H,11H-azeto[2,1-c]imidazo[1,5-a][1,4]benzodiazepine-1-carboxylate.

## Patentansprüche (Patentansprüche für folgende(n) Vertragsstaat(en): AT, BE, CH, DE, FR, GB, GR, IT, LI, LU, NL, SE)

1. Verwendung einer Benzodiazepinverbindung mit der Formel 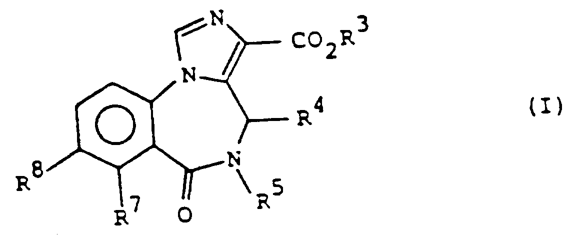
worin R³ Wasserstoff bedeutet, R⁷ und R⁸ unabhängig voneinander Wasserstoff, Halogen, CF₃, CN, NO₂, NH₂, C₁₋₄-Alkyl oder C₁₋₄-Alkoxy bedeuten; und R⁴ Wasserstoff und R⁵ Wasserstoff oder C₁₋₇-Alkyl bedeutet; oder R⁴ und R⁵ zusammen (CH₂)ₙ bedeuten, wobei n eine ganze Zahl von 2 bis 3 ist, als aktiver Bestandteil zur Herstellung einer pharmazeutischen Zusammensetzung zur Behandlung einer Störung des zentralen Nervenystems, reagierend auf Antagonisierung von erregenden Aminosäuren, die über die Quisqualat- oder Kainat-Rezeptoren wirken.

2. Verwendung gemäß Anspruch 1, worin der aktive Bestandteil 8-Fluor-5,6-dihydro-5-methyl-6-oxo-4H-imidazo[1,5-a][1,4]benzodiazepin-3-carboxylat ist.

3. Verwendung gemäß Anspruch 1, worin der aktive Bestandteil 8-Brom-11,12,13,13a-tetrahydro-9-oxo-9H-imidazo[1,5-a]pyrrolo[2,1-c][1,4]benzodiazepin-1-carboxylat ist.

4. Verwendung gemäß Anspruch 1, worin der aktive Bestandteil 12,12a-Dihydro-8-chlor-9-oxo-9H,11H-azeto[2,1-c]imidazo[1,5-a][1,4]benzodiazepin-1-carboxylat ist.

## Patentansprüche (Patentansprüche für folgende(n) Vertragsstaat(en): ES)

1. Verfahren zur Herstellung einer pharmazeutischen Zusammensetzung für die Behandlung einer Störung des zentralen Nervensystems, reagierend auf eine Antagonisierung von erregenden Aminosäuren, die über Quisqualat- oder Kainat-Rezeptoren wirken, umfassend den Schritt der Kombination der Verbindung mit der Formel: 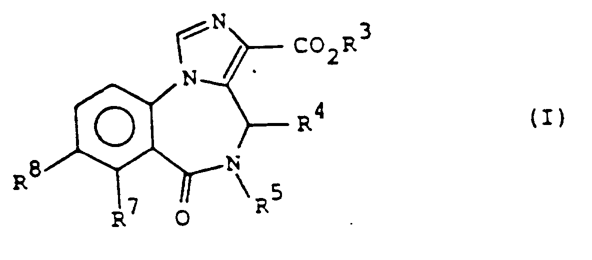
in der R³ Wasserstoff bedeutet
R⁷ und R⁸ unabhängig voneinander Wasserstoff, Halogen, CF₃, CN, NO₂, NH₂, C₁₋₄-Alkyl oder C₁₋₄-Alkoxy bedeuten; und
R⁴ Wasserstoff und R⁵ Wasserstoff oder C₁₋₇-Alkyl bedeutet; oder R⁴ und R⁵ zusammen (CH₂)ₙ bedeuten, wobei n eine ganze Zahl von 2-3 ist, mit einem konventionellen Hilfsmittel, Träger oder Verdünnungsmittel in Form einer pharmazeutischen Zusammensetzung oder ihrer Einheitsdosis.

2. Verfahren gemäß Anspruch 1, worin der aktive Bestandteil 8-Fluor-5,6-dihydro-5-methyl-6-oxo-4H-imidazo[1,5-a][1,4]benzodiazepin-3-carboxylat ist.

3. Verfahren gemäß Anspruch 1, worin der aktive Bestandteil 8-Brom-11,12,13,13a-tetrahydro-9-oxo-9H-imidazo[1,5-a]pyrrolo[2,1-c][1,4]benzodiazepin-1-carboxylat ist.

4. Verfahren gemäß Anspruch 1, worin der aktive Bestandteil 12,12a-Dihydro-8-chlor-9-oxo-9H,11H-azeto[2,1-c]imidazo[1,5-a][1,4]benzodiazepin-1-carboxylat ist.

## Revendications (Revendications pour l'(les) Etat(s) contractant(s) suivant(s): AT, BE, CH, DE, FR, GB, GR, IT, LI, LU, NL, SE)

1. Utilisation d'une benzodiazépine ayant la formule 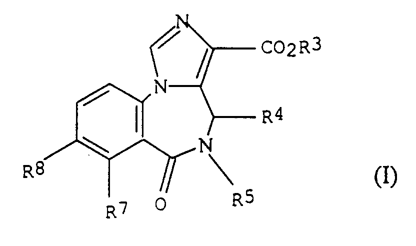 dans laquelle
R³ est un atome d'hydrogène
R⁷ et R⁸ sont indépendamment un atome d'hydrogène, un halogène, ou un groupe CF₃, CN, NO₂, NH₂, alkyle en C₁₋₄ ou alcoxy en C₁₋₄ ; et
R⁴ est un atome d'hydrogène et R⁵ est un atome d'hydrogène ou un groupe alkyle en C₁₋₇ ; ou R⁴ et R⁵ désignent ensemble le groupe (CH₂)ₙ dans lequel n est un nombre entier de 2-3, comme ingrédient actif pour la préparation d'une composition pharmaceutique pour le traitement des troubles du système nerveux central sensibles à l'antagonisation des acides aminés excitateurs agissant via les récepteurs du quisqualate ou du kainate.

2. Utilisation selon la revendication 1, dans laquelle l'ingrédient actif est le 3-carboxylate de 8-fluoro-5,6-dihydro-5-méthyl-6-oxo-4H-imidazo[1,5-a][1,4]benzodiazépine.

3. Utilisation selon la revendication 1, dans laquelle l'ingrédient actif est le 1-carboxylate de 8-bromo-11,12,13,13a-tétrahydro-9-oxo-9H-imidazo[1,5-a]-pyrrolo[2,1-c][1,4]benzodiazépine.

4. Utilisation selon la revendication 1, dans laquelle l'ingrédient actif est le 1-carboxylate de 12,12a-dihydro-8-chloro-9-oxo-9H,11H-azéto[2,1-c]-imidazo[1,5-a][1,4]benzodiazépine.

## Revendications (Revendications pour l'(les) Etat(s) contractant(s) suivant(s): ES)

1. Procédé de préparation d'une composition pharmaceutique pour le traitement des troubles du système nerveux central sensibles à l'antagonisation d'acides aminés excitateurs agissant via les récepteurs du quisqualate ou du kainate, comprenant l'étape consistant à mettre le composé ayant la formule : 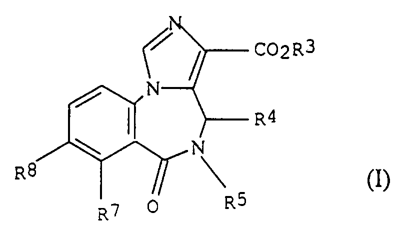 dans laquelle
R³ est un atome d'hydrogène
R⁷ et R⁸ sont indépendamment un atome d'hydrogène, un halogène, ou un groupe CF₃, CN, NO₂, NH₂, alkyle en C₁₋₄ ou alcoxyle en C₁₋₄ ; et
R⁴ est un atome d'hydrogène et R⁵ est un atome d'hydrogène ou un groupe alkyle en C₁₋₇ ; ou R⁴ et R⁵ désignent ensemble le groupe (CH₂)ₙ dans lequel n est un nombre entier de 2-3, en association avec un adjuvant, un porteur ou un diluant classique sous forme de composition pharmaceutique ou de dose unitaire de celle-ci.

2. Procédé selon la revendication 1, dans lequel l'ingrédient actif est le 3-carboxylate de 8-fluoro-5,6-dihydro-5-méthyl-6-oxo-4H-imidazo[1,5-a]-[1,4]benzodiazépine.

3. Procédé selon la revendication 1, dans lequel l'ingrédient actif est le 1-carboxylate de 8-bromo-11,12,13,13a-tétrahydro-9-oxo-9H-imidazo[1,5-a]pyrrolo[2,1-c][1,4]benzodiazépine.

4. Procédé selon la revendication 1, dans lequel l'ingrédient actif est le 1-carboxylate de 12,12a-dihydro-8-chloro-9-oxo-9H,11H-azéto[2,1-c]imidazo [1,5-a][1,4]benzodiazépine.
